(19) Europäisches Patentamt
European Patent Office
Office européen des brevets

(11) EP 0 811 618 B1

(12) EUROPÄISCHE PATENTSCHRIFT

(45) Veröffentlichungstag und Bekanntmachung des Hinweises auf die Patenterteilung:
**23.10.2002 Patentblatt 2002/43**

(51) Int Cl.[7]: **C07D 307/46**, C07D 307/38, A23L 1/234

(21) Anmeldenummer: **97108874.5**

(22) Anmeldetag: **03.06.1997**

(54) **Furfurylthioalkane, Verfahren zu ihrer Herstellung und ihre Verwendung**

Furfurylthioalkanes, process for their preparation and use thereof

Furfurylthioalkanes, procédé pour leur préparation et leur utilisation

(84) Benannte Vertragsstaaten:
**CH DE FR GB IT LI NL**

(30) Priorität: **07.06.1996 DE 19622745**

(43) Veröffentlichungstag der Anmeldung:
**10.12.1997 Patentblatt 1997/50**

(73) Patentinhaber: **HAARMANN & REIMER GMBH**
**D-37601 Holzminden (DE)**

(72) Erfinder:
- **Bertram, Heinz Jürgen Dr.**
  **Teterboro, N.J. 07608 (US)**
- **Werkhoff, Peter Dr.**
  **37671 Höxter (DE)**
- **Güntert, Matthias Dr.**
  **37603 Holzminden (DE)**

(74) Vertreter: **Zobel, Manfred, Dr. et al**
**BAYER AG**
**Konzernbereich RP**
**Patente und Lizenzen**
**51368 Leverkusen (DE)**

(56) Entgegenhaltungen:
**US-A- 3 952 024**
**US-A- 3 952 026**
**US-A- 4 092 334**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 811 618 B1

Printed by Jouve, 75001 PARIS (FR)

## Beschreibung

Die Erfindung betrifft neue Furfurylthioalkane, ein Verfahren zu ihrer Herstellung und ihre Verwendung als Aromastoffe.

Es wurde gefunden, daß ausgewählte Furfurylthioalkane wertvolle organoleptische Eigenschaften besitzen.

Gegenstand der Erfindung sind Verbindungen der Formel

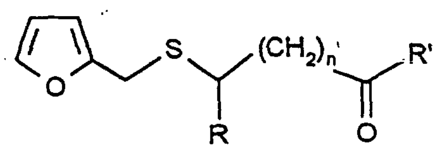

(I)

worin

n Null oder 1 sein kann,

R für den Methyl- oder Ethylrest steht, wenn n = ist, und für Wasserstoff oder den Methylrest steht, wenn n = 1 ist, und

R' für Methyl oder Ethyl steht.

Bevorzugte Verbindungen I entsprechen den Formeln

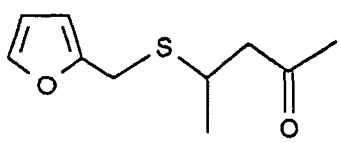

(Ia)

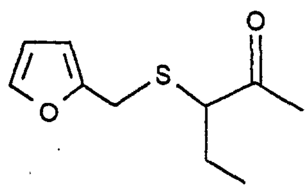

(Ib)

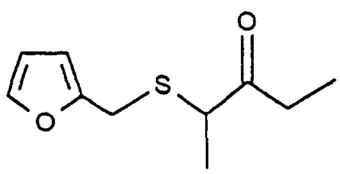

(Ic)

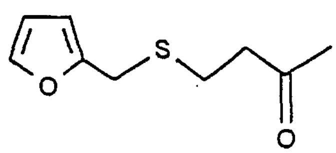

(Id)

Die Verbindungen I können durch Umsetzung von Furfurylmercaptan mit Ketoverbindungen hergestellt werden.

Weiterer Gegenstand der Erfindung ist daher ein Verfahren zur Herstellung der Verbindungen I durch Umsetzung von Furfurylmercaptan mit Ketonen der Formeln

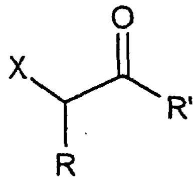

(II)

worin

X für Halogen, vorzugsweise Chlor oder Brom, und

R für Methyl oder Ethyl stehen, oder

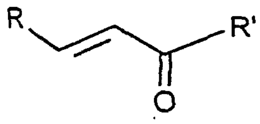

(III)

worin R für Wasserstoff oder Methyl steht, wobei R' jeweils die oben angegebene Bedeutung besitzt.

Bevorzugte Ketone II sind beispielsweise 3-Chlor-butan-2-on, 3-Brom-butan-2-on, 3-Chlorpentan-2-on, 3-Brompentan-2-on, bevorzugte Ketone III sind beispielsweise But-3-en-2-on und Pent-3-en-2-on.

Das Verfahren kann in Substanz, vorzugsweise aber in inerten organischen Lösungsmittein durchgeführt werden. Bevorzugte organische Lösungsmittel umfassen Alkohole, wie Ethanol, und Ether, wie Methyl-tert.-butylether und Tetrahydrofuran.

Die Umsetzung von Furfurylmercaptan mit Keton II wird man in Gegenwart einer Base vornehmen, um den freiwerdenden Halogenwasserstoff zu binden. Es wird bevorzugt, mindestens äquivalente Mengen Base einzusetzen. Auch die Umsetzung von Furfurylmercaptan mit Keton III kann man durch Basenzusatz katalysieren. Während auch hier oft äquivalente Mengen zu besten Ergebnissen führen, können sehr oft auch sehr geringe Mengen ausreichen; durch einige wenige Versuche läßt sich die optimale Menge leicht feststellen.

Bevorzugte Basen umfassen beispielsweise Amine wie Triethylamin, Morpholin, Piperidin, Pyridin, Alkoholate wie Natriummethylat und Kalium-tert.-butylat und Hydroxide wie Natrium- und Kaliumhydroxid.

Das Verfahren läßt sich innerhalb eines weiteren Temperaturbereiches durchführen. Im allgemeinen sind Temperaturen von -30 bis +50, insbesondere von 0 bis 30°C bevorzugt.

Die erfmdungsgemäßen Verbindungen (I) sind wertvolle Aromastoffe; sie zeichnen sich durch sehr niedrige Geschmacksschwellenwerte aus. So fand ein Testpanel aus 20 Prüfern in einem Dreieckstest bei der Anwendung von 4-Furylthio-butan-2-on in 0,5 gew.-%iger wäßriger Kochsalzlösung bereits einen signifikanten Unterschied zwischen der Null-Probe und der nur 1 ppb 4-Furfurylthio-butan-2-on enthaltenden Kochsalzlösung. Für 3-Furfurylthio-pentan-2-on fand ein Testpanel aus 6 geschulten Prüfern einen signifikanten Unterschied zwichen der 0,5 gew.-%igen Kochsalzlösung und der 1,5 ppb 3-Furfurylthiopentan-2-on enthaltenden 0,5 gew.-%igen Kochsalzlösung.

Die Geschmacksbeschreibungen für die einzelnen erfindungsgemäßen Verbindungen der Formel (I) bei ihrer Anwendung in 0,5 gew.-%iger wäßriger Kochsalzlösung lauten:

4-Furfurylthio-butan-2-on:

bei einem Zusatz von 1 ppb: brandig, zwieblig, Kaffeenote, röstig, Mokka, schweflig

4-Furfurylthio-pentan-2-on:

bei einem Zusatz von 1 ppb: Kaffee, Mokka, röstig

3 -Furfurylthio-pentan-2-on:

bei einem Zusatz von 0,6 ppm: Kaffee, röstig, Zartbitterschokolade, Mokka

3-Furfurylthio-butan-2-on:

bei einem Zusatz von 150 ppb: röstig, schweflig, Kaffee, leicht Mokka

Mit ihrem spezifischen Geschmack in Richtung Mokka wirken die erfindungsgemäßen Verbindungen (I) in Kaffee- und Mokkakompositionen geschmacksverstärkend und -abrundend. Aber auch in anderen Aromakompositionen, z.B. Nußaromen, bewirken die erfindungsgemäßen Verbindungen eine Abrundung des Aromas und eine Erhöhung der Geschmacksfülle.

Die unter Verwendung der erfindungsgemäßen Verbindungen hergestellten Aromakompositionen können im gesamten Nahrungs- und Genußmittelbereich sowie in Tierfutter eingesetzt werden. Insbesondere sind sie für Fettmassen, Backwaren, Extrusionsprodukte, Fertiggerichte, Fleisch- und Wurstprodukte, Suppen, Soßen, Gemüsekonserven und alle Artem von industriell gefertigten Tierfutter geeignet.

Die erfindungsgemäßen, neuen Furfuryl-thioalkane werden in der Regel in Mengen von 5 ppt bis 1 Gew.-%, vorzugsweise 100 ppt bis 100 ppm, bezogen auf das verzehrfertige Nahrungsmittel, verwendet.

Weiterer Gegenstand der Erfindung ist also die Verwendung der Verbindungen I als Aromastoffe.

Bei den Prozentangaben der nachfolgenden Beispiele handelt es sich um Gewichtsprozente.

## Beispiele

### Beispiel 1

4-Furfurylthio-pentan-2-on

10 g Furfurylmercaptan und 15 g 3-Penten-(2)-on wurden in 100 ml Ethanol gelöst. Die Lösung wurde 48 Stunden bei Raumtemperatur gerührt. Anschließend wurde das Lösungsmittel bei 50°C im Vakuum abdestilliert. Der Rückstand (18 g) wurde durch präparative Hochdruckflüssigkeitschromatographie (HPLC) gereinigt. Es wurden 9 g 4-Furfurylthio-pentan-2-on (Reinheitsgrad 95 %) erhalten. IR-, NMR-und Massenspektren der Verbindung stimmen mit der für sie angegebenen Struktur überein.
IR-Spektrum (Film):

| Wellenzahl [$cm^{-1}$] | |
|---|---|
| 745.2 | m |
| 935.3 | m |
| 1010.9 | m |
| 1159.9 | m |
| 1361.9 | m |
| 1420.7 | m |
| 1504.5 | m |
| 1711.5 | s |
| 2925.6 | w |
| 2966.8 | m |
| (Intensität der IR-Banden: w = schwach, m = mittel, s = stark) | |

### Beispiel 2

Analog Beisoiel 1 wurde bei Verwendung von 3-Buten-(2)-on anstelle von 3-Penten-(2)-on 4-Furfurylthio-butan-2-on erhalten.

### Beispiel 3

3-Furfurylthio-pentan-2-on

Man legt 11,2 g Kalium-tert.-butylat, gelöst in 100 ml trockenem Tetrahydrofuran bei Raumtemperatur vor. Hierzu tropft man bei 20°C 11,4 g Furfurylmercaptan. Nach vollständiger Zugabe wird für weitere 30 Minuten nachgerührt. Dann tropft man 16,5 g 3-Brompentan-2-on im Verlauf von 30 Minuten zu und rührt für eine Stunde nach. Zur

Aufarbeitung wird der Ansatz auf 100 ml Wasser gegeben, zweimal mit Diethylether extrahiert, die vereinigten organischen Phasen mit Wasser gewaschen, über Natriumsulfat getrocknet und eingeengt. Es werden 19 g Rohprodukt (Reinheit 84,1 %) erhalten. Zur weiteren Reinigung unterwirft man das Produkt einer Kugelrohrdestillation. Es werden 10 g 3-Furfurylthio-pentan-2-on erhalten, das eine Reinheit von 97,5 % aufweist. IR-, NMR- und Massenspektren der Verbindung stimmen mit der für sie angegebenen Struktur überein.
IR-Spektrum (Film):

| Wellenzahl [$cm^{-1}$] | |
|---|---|
| 747.1 | m |
| 936.6 | m |
| 1010.8 | m |
| 1153.2 | m |
| 1213.9 | w |
| 1355.7 | m |
| 1417.6 | w |
| 1504 | m |
| 1695.4 | s |
| 2967.4 | m |

**Beispiel 4**

Analog Beispiel 3 wurde bei Verwendung von 3-Brombutan-2-on anstelle von 3-Brompentan-2-on 3-Furfurylthiobutan-2-on erhalten.

**Beispiel 5**

Analog Beispiel 3 wurde bei Verwendung von 2-Brompentan-3-on anstelle von 3-Brompentan-2-on 2-Furfurylthio-3-pentanon erhalten.
IR-Spektrum (Film):

| Wellenzahl [$cm^{-1}$] | |
|---|---|
| 746,3 | m |
| 936,6 | m |
| 1011,4 | m |
| 1152,6 | m |
| 1353,2 | m |
| 1455,1 | m |
| 1504,1 | m |
| 1696,1 | s |
| 2936,3 | m |
| 2975,1 | m |

**Anwendung**

Eine Kaffeekomposition wurde durch Mischen folgender Bestandteile in den angegebenen Gewichtsteilen hergestellt:

| | Gewichtsteile |
|---|---|
| 3,5(6)-Dimethyl-2-ethylpyrazin | 20 |
| Diacetyl | 20 |
| Isobutyraldehyd | 20 |
| 3-Methyl-2-cyclopentan-2-ol-1-on | 50 |
| 2,6-Dimethoxyphenol | 50 |

(fortgesetzt)

| | Gewichtsteile |
|---|---|
| Capronsäure | 100 |
| 2,5-Dimethyl-4-hydroxyfuran-3(2H)-on | 30 |
| erfindungsgem. Furfurylthioalkan | 10-100 |
| Triacetin | 610-700 |
| | 1000 |

**Patentansprüche**

**1.** Verbindungen der Formel

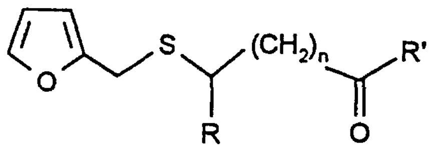
(I)

worin

n Null oder 1 sein kann,

R für den Methyl- oder Ethylrest steht, wenn n = 0 ist, und für Wasserstoff oder den Methylrest steht, wenn n = 1 ist, und

R' für Methyl oder Ethyl steht.

**2.** Verbindungen nach Anspruch 1, ausgewählt aus der Reihe
3-Furfurylthiopentan-2-on,
4-Furfurylthiopentan-2-on,
4-Furfurylthiobutan-2-on,
4-Furfurylthiopentan-3-on.

**3.** Verfahren zur Herstellung der Verbindungen nach Anspruch 1 durch Umsetzung von Furfurylmercaptan mit Ketonen der Formeln

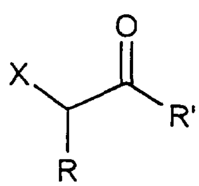
(II)

worin

X für Halogen und

R für Methyl oder Ethyl stehen, oder

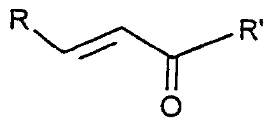

(III)

worin R für Wasserstoff oder Methyl steht, wobei R' jeweils die in Anspruch 1 angegebene Bedeutung besitzt.

**4.** Verwendung der Verbindungen nach Anspruch 1 als Aromastoffe.

**Claims**

**1.** Compounds of formula

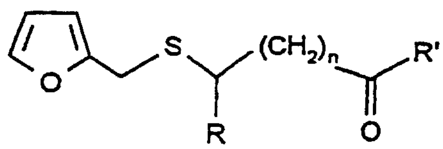

(I)

wherein

n can be zero or 1,

R represents a methyl or ethyl radical if n = 0, and represents hydrogen or a methyl radical if n = 1, and

R' represents methyl or ethyl.

**2.** Compounds according to claim 1, selected from the series
3-furfurylthiopentan-2-one,
4-furfurylthiopentan-2-one,
4-furfurylthiobutan-2-one,
4-furfurylthiopentan-3-one.

**3.** A method of producing compounds according to claim 1 by the reaction of furfurylmercaptan with ketones of formulae

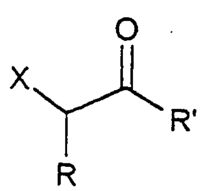

(II)

wherein

X represents a halogen and

R represents methyl or ethyl, or

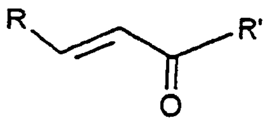 (III)

wherein R represents hydrogen or methyl, and wherein R' has the meaning given in claim 1 in each case.

**4.** Use of the compounds according to claim 1 as flavourings.

**Revendications**

**1.** Composés de la formule

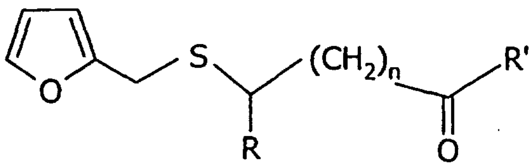 (I)

où

n peut être égal à 0 ou à 1,
R représente le reste méthyle ou éthyle lorsque n = 0, et représente l'atome d'hydrogène ou le reste méthyle lorsque n = 1, et
R' représente le groupe méthyle ou éthyle.

**2.** Composés selon la revendication 1, choisis parmi
la 3-furfurylthiopentan-2-one,
la 4-furfurylthiopentan-2-one,
la 4-furfurylthiobutan-2-one,
la 4-furfurylthiopentan-3-one.

**3.** Procédé pour la préparation des composés selon la revendication 1 par réaction de furfurylmercaptan avec des cétones des formules

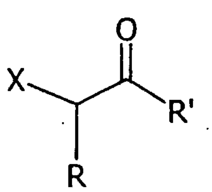 (II)

où

X représente un atome d'halogène et
R représente le groupe méthyle ou éthyle, ou

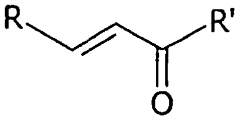

(III)

où R représente l'atome d'hydrogène ou le groupe méthyle, R' ayant à chaque fois la signification indiquée dans la revendication 1.

**4.** Utilisation des composés selon la revendication 1 comme matières d'arômes.